(19) Europäisches Patentamt / European Patent Office / Office européen des brevets

(11) **EP 4 233 687 A1**

(12) **EUROPEAN PATENT APPLICATION**
published in accordance with Art. 153(4) EPC

(43) Date of publication:
**30.08.2023 Bulletin 2023/35**

(21) Application number: **21882774.9**

(22) Date of filing: **18.10.2021**

(51) International Patent Classification (IPC):
***A61B 3/028*** (2006.01)

(52) Cooperative Patent Classification (CPC):
**A61B 3/028**

(86) International application number:
**PCT/JP2021/038456**

(87) International publication number:
**WO 2022/085633 (28.04.2022 Gazette 2022/17)**

(84) Designated Contracting States:
**AL AT BE BG CH CY CZ DE DK EE ES FI FR GB
GR HR HU IE IS IT LI LT LU LV MC MK MT NL NO
PL PT RO RS SE SI SK SM TR**
Designated Extension States:
**BA ME**
Designated Validation States:
**KH MA MD TN**

(30) Priority: **22.10.2020 JP 2020177324**

(71) Applicants:
• **Mitsui Chemicals, Inc.**
  **Tokyo 104-0028 (JP)**
• **Visual Technology Laboratory Inc.**
  **Tokyo 140-0004 (JP)**

(72) Inventors:
• **ITO, Daiki**
  **Sodegaura-shi, Chiba 299-0265 (JP)**

• **NISHIMOTO, Taizo**
  **Sodegaura-shi, Chiba 299-0265 (JP)**
• **SUZUKI, Kenji**
  **Sodegaura-shi, Chiba 299-0265 (JP)**
• **MATSUMURA, Shoko**
  **Sodegaura-shi, Chiba 299-0265 (JP)**
• **ASADA, Noriaki**
  **Tokyo 104-0028 (JP)**
• **NAKAMURA, Yoshiki**
  **Tokyo 140-0004 (JP)**
• **YAMAMOTO, Minao**
  **Tokyo 140-0004 (JP)**

(74) Representative: **Mewburn Ellis LLP**
**Aurora Building
Counterslip
Bristol BS1 6BX (GB)**

(54) **INFORMATION PROCESSING DEVICE, INFORMATION PROCESSING METHOD, AND COMPUTER PROGRAM**

(57) An information processing device 10 including a calculation unit 101 that calculates an evaluation function using a visibility threshold of a test subject obtained by performing a visual acuity examination on the test subject and using a parameter related to a visual object, and a spectral transmittance optimization unit 102 that optimizes a spectral transmittance of a filter having a spectral transmittance to light from the visual object using the evaluation function when passed through the filter.

FIG.3

EP 4 233 687 A1

**Description**

Technical Field

The present disclosure relates to an information processing device, an information processing method, and a computer program.

Background Art

An optical member such as a lens for a pair of glasses has optical characteristics such as spectral transmittance. The spectral transmittance of a lens for a pair of glasses changes a spectral distribution of light entering the eye, and so when a visual object is looked at by a person through a lens for a pair of glasses, the luminance and color thereof is changed. Color is, for example, expressed by tristimulus values in CIE XYZ color space. An optical member can be designed to match each individual by calculating the optical characteristics for a vision function of each individual based on objective quantitative evaluation. There is accordingly a proposal for technology capable of performing objective quantitative evaluation for each individual.

A vision function examination system disclosed in Patent Document 1 includes an analysis means to find at least two boundaries by finding a boundary between a luminance contrast region where a visual target is visible to a test subject and a luminance contrast region where the visual target is not visible to the test subject for each combination of stimuli values of color stimuli of a visual target in a coordinate system indicating a correlation between a luminance contrast value between a luminance of an attention portion and a luminance of a background portion, and a luminance average value of the visual target including the attention portion and the background portion.

Related Art

Related Patent Documents

Patent Document 1: International Publication (WO) No. 2018/012334

SUMMARY OF INVENTION

Technical Problem

In the technology disclosed in Patent Document 1, although a visual acuity examination is performed on a test subject and a vision function of the test subject can be quantified thereby, objective quantitative evaluation is not performed in consideration of a combination of information about the visual acuity of the test subject and information about a visual object.

In consideration of the above circumstances, an object of the present disclosure is to provide an information processing device, an information processing method, and a computer program that enable an optimal optical member to be designed for each test subject in consideration of a combination of information about the visual acuity of the test subject and information about a visual object.

Solution to Problem

In order to achieve the above object, an information processing device according to the present disclosure includes a calculation unit that calculates an evaluation function using a visibility threshold of a test subject, obtained by performing a visual acuity examination on the test subject, and using a parameter related to a visual object, and a spectral transmittance optimization unit that optimizes a spectral transmittance of a filter having a specific spectral transmittance to light from the visual object using the evaluation function when passed through the filter.

The spectral transmittance optimization unit may be configured to determine the spectral transmittance of the filter such that a value of the evaluation function is maximized.

The spectral transmittance optimization unit may be configured to determine the spectral transmittance such that the value of the evaluation function is maximized from among spectral transmittances generated by combining pre-prepared dyes.

The spectral transmittance optimization unit may be configured to determine a spectral transmittance such that a value of the evaluation function is maximized using a specific numerical optimization algorithm.

The calculation unit may be configured to calculate the evaluation function for a specified part region of the visual object.

The calculation unit may be configured to calculate the evaluation function for a part region of the visual object as specified by a result of performing edge detection on the visual object.

The spectral transmittance optimization unit may be configured to optimize the spectral transmittance of the filter using the evaluation function when passed through the filter at a point in the region where a value of the evaluation function when not passed through the filter is maximized.

The spectral transmittance optimization unit may be configured to, in a case in which a first point that is a point in the region where the value of the evaluation function when not passed through the filter is maximized differs from a second point that is a point in the region where a value of the evaluation function when passed through the filter is maximized, optimize the spectral transmittance of the filter using the evaluation function at the first point.

The visibility threshold of the test subject may be a luminance contrast visibility threshold.

The luminance contrast visibility threshold may be generated by finding a boundary between a luminance contrast region where a visual target is visible to the test subject and a luminance contrast region where the visual target is not visible to the test subject in a coordinate system indicating a correlation between a luminance contrast value between a luminance of an attention portion and a luminance of a background portion, and a luminance average value of the visual target including the attention portion and the background portion.

The boundary may be generated by finding a visibility function that approximates to the one which takes visibility changes into account due to superposition of color of visual targets in the coordinate system.

The parameter related to the visual object may be a luminance contrast of the visual object calculated from spectral radiance of the visual object.

Moreover, in order to achieve the above object, an information processing method according to the present disclosure is a method that is executed by a computer. The method includes calculating an evaluation function using a visibility threshold of a test subject obtained by performing a visual acuity examination on the test subject and using a parameter related to a visual object, and optimizing a spectral transmittance of a filter having a spectral transmittance to light from the visual object using the evaluation function when passed through the filter.

Moreover, in order to achieve the above object, a computer program according to the present disclosure causes a computer to execute processing. The processing includes calculating an evaluation function using a luminance contrast visibility threshold of a test subject obtained by performing a visual acuity examination on the test subject and using a parameter related to a visual object, and optimizing a spectral transmittance of a filter having a spectral transmittance to light from the visual object using the evaluation function when passed through the filter.

Advantageous Effects

The present disclosure thereby enables provision of an information processing device, an information processing method, and a computer program that enable an optimal optical member to be designed for each test subject in consideration of a combination of information about the visual acuity of the test subject and information about a visual object by using an evaluation function derivable from a visibility threshold of the test subject and a parameter related to a visual object.

BRIEF DESCRIPTION OF DRAWINGS

Fig. 1 is a diagram illustrating a schematic configuration of an optical member design system that includes an information processing device according to an exemplary embodiment.
Fig. 2 is a block diagram illustrating a hardware configuration of an information processing device.
Fig. 3 is a block diagram illustrating an example of a functional configuration of an information processing device.
Fig. 4 is a diagram illustrating an example of a luminance contrast threshold data of a test subject.
Fig. 5 is a flowchart illustrating a flow of optical member design processing by an information processing device.
Fig. 6 is a diagram to explain calculation processing of an evaluation function by a CPU.
Fig. 7 is a diagram to explain calculation processing of an evaluation function by a CPU.

DESCRIPTION OF EMBODIMENTS

Following description is regarding an example of an exemplary embodiment of the present disclosure, with reference to the drawings. Note that configuration element and parts that are the same or equivalent in the drawings are appended with the same reference numerals. Moreover, sometimes dimensional proportions have been exaggerated in the drawings for ease of explanation, and differ from actual proportions.

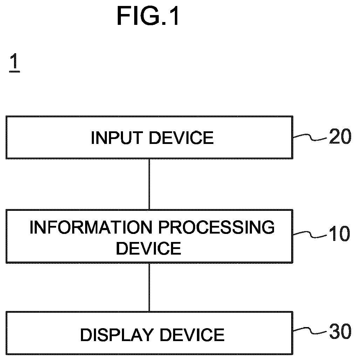

Fig. 1 is a diagram illustrating a schematic configuration of an optical member design system including an information processing device according to the present exemplary embodiment. The optical member design system 1 illustrated in Fig. 1 includes an information processing device 10, an input device 20, and a display device 30.

The information processing device 10 is a device for designing an optimal optical member for each test subject by calculating an evaluation value based on a vision function of the test subject as quantitatively evaluated by a visual acuity examination of the test subject, and based on information about a visual object. The visual acuity examination of the test subject may, for example, be performed using the technology disclosed in International Publication (WO) No. 2018/012334. The information processing device 10 may hold information about a visual acuity examination of the test subject as performed using the technology disclosed in WO No. 2018/012334, and may hold information about a visual acuity examination of the test subject as performed using such technology with a different device to the information processing device 10.

The input device 20 includes a pointing device such as a mouse and a keyboard, and is employed to perform various inputs. The input device 20 is employed in optical member design processing by the information processing device 10, and is employed to input spectral radiance information of an image. The spectral radiance information of images is obtained by measuring a given visual object using a spectral radiance meter. A two-dimensional spectral radiance meter may be employed as the spectral radiance meter. Imaging the visual object using a two-dimensional spectral radiance meter enables spectral radiance information for each pixel of a specific image size to be obtained as an image. Moreover, point measurement values for a given visual object may be collected using a spectral radiance meter to obtain luminance distribution information of the visual object.

The display device 30 is, for example, a liquid crystal display or an organic EL display, and displays various information. The display device 30 may employ a touch panel so as to also function as the input device 20. The display device 30 is employed during a vision function examination of the test subject, and is moreover also employed to display information when designing an optimal optical member for each test subject.

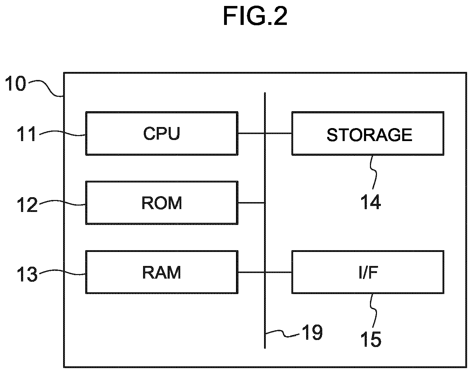

Fig. 2 is a block diagram illustrating a hardware configuration of the information processing device 10.

As illustrated in Fig. 2, the information processing device 10 includes a central processing unit (CPU) 11, read only memory (ROM) 12, random access memory (RAM) 13, storage 14, and an interface (I/F) 15. Each configuration is connected together through a bus 19 so as to be capable of communicating with each other.

The CPU 11 is a central processing unit that executes various programs and controls each section. Namely, the CPU 11 reads a program from the ROM 12 or the storage 14, and executes the program using the RAM 13 as workspace. The CPU 11 controls each of the above configuration and performs various calculation processing according to the program recorded on the ROM 12 of the storage 14. In the present exemplary embodiment there is an optical member design program for designing an optimal optical member for each test subject stored on the ROM 12 or the storage 14.

The ROM 12 is stored with various programs and various data. The RAM 13 serves as workspace to temporarily store a program or data. The storage 14 is configured by a storage device such as a hard disk drive (HDD), solid state drive (SSD), or flash memory, and is stored with various programs including an operating system, and various data.

The interface 15 is an interface for exchanging signals from other devices, such as the input device 20 or the display device 30, and employs, for example, a standard such as universal serial bus (USB), DVI-D, DVI-I, High Definition Multimedia Interface (HDMI, registered trademark), Ethernet (registered trademark), FDDI, Wi-Fi (registered trademark), or the like.

The information processing device 10 implements various functions when executing the above design program using the hardware resources described above. Explanation follows regarding functional configuration implemented by the information processing device 10.

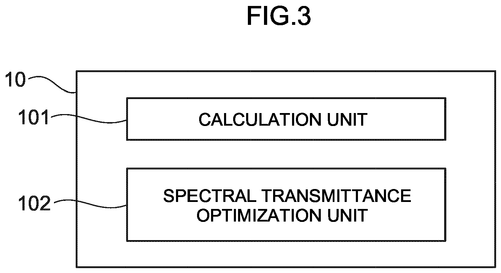

Fig. 3 is a block diagram illustrating an example of a functional configuration of the information processing device 10.

As illustrated in Fig. 3, the information processing device 10 includes, as functional configuration, a calculation unit 101, and a spectral transmittance optimization unit 102. Each of the functional configuration is implemented by the CPU 11 reading and executing the optical member design program stored in the ROM 12 or the storage 14.

The calculation unit 101 calculates an evaluation value from an evaluation function based on visibility threshold data of a test subject obtained by performing a visual acuity examination on the test subject and based on two-dimensional spectral radiance image data of a visual object. The visual object may be pixels in an image. The calculation unit 101 calculates a value of an evaluation function $F_{VIS}$ (hereafter referred to as an evaluation value) as defined by the following Equation (1), wherein $LC_{th}$ is a luminance contrast visibility threshold calculated from visibility threshold data of a test subject, and $LC_{obj}$ is a luminance contrast calculated from two-dimensional spectral radiance image data of a visual object. The evaluation value is an index in which the larger the value, the more easily the visual object is seen by the test subject. Namely, the evaluation value becomes larger by the denominator $LC_{th}$ being smaller and the numerator $LC_{obj}$ being larger. In particular, an evaluation value of larger than 1 means that the visual object is visible the test subject, and an evaluation value of 1 or lower means that the visual object is not visible to the test subject. An objective of the optical member design program is to calculate a spectral transmittance of a lens for a pair of glasses (hereafter referred to as a filter) so as to raise the ease with which the visual object is seen by the test subject, by calculation using the evaluation function. Note that Equation (1) is disclosed as Visibility Level in "Study on visibility evaluation of circular visual targets for sighted subjects - research on visibility evaluation method using a luminance contrast evaluation graph assuming subjects with poor vision", Part 2 by Yoko KATO, Yoshiki NAKAMURA and Michico IWATA in Journal of Japanese Environmental Engineering Vol. 83, No. 749, pp. 565-572, July 2018.

$$F_{VIS} = LC_{obj}/LC_{th} \qquad (1)$$

The visual acuity examination on the test subject may be performed using, for example, the technology disclosed in WO No. 2018/012334. The visibility threshold data of the test subject is obtained as a borderline luminance between a luminance of a visual target that was visible and a luminance of a visual target that was not visible, for example as disclosed in WO No. 2018/012334. Moreover, the two-dimensional spectral radiance image data of the visual object is obtained by measuring a given visual object at a specific measurement angle (for example 1°) using a spectral radiance meter.

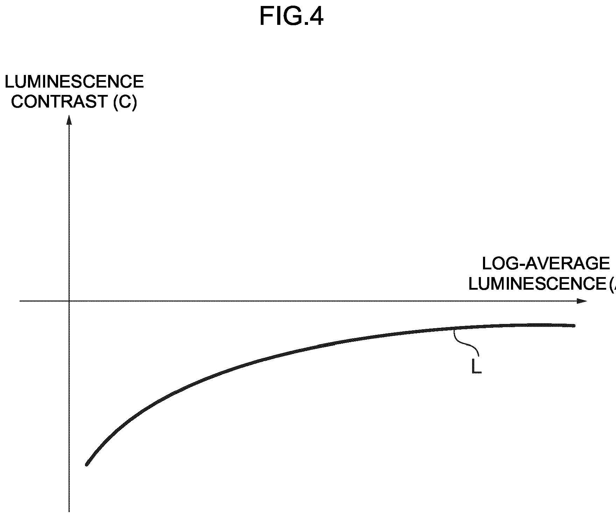

Fig. 4 is a diagram illustrating an example of luminance contrast visibility thresholds for a test subject. The luminance contrast is a value indicating a contrast in luminance of an attention portion of a visual target with respect to a background portion thereof. In particular, a luminance contrast of a visibility threshold is called a luminance contrast visibility threshold. A luminance average is a value indicating an average of luminance of a visual target, including the attention portion and the background portion thereof. In the present exemplary embodiment, a log-average luminance is employed as an example of a method for calculating the luminance average. Note that the luminance contrast and the luminance average may be calculated by any calculation method. A line L illustrated in Fig. 4 is a visibility threshold curve obtained by plotting luminance contrast visibility threshold values of the test subject for each successive change in log-average luminance of the visual target. Note that the luminance contrast C on the vertical access is a positive value in cases in which the attention portion of the visual target has a higher luminance than the background portion thereof.

A basis under which a visibility threshold curve L is defined is research disclosed in "Study on visibility evaluation of circular visual targets for sighted subjects - research on visibility evaluation method using a luminance contrast evaluation graph assuming subjects with poor vision, Part 1" by Yoko KATO, Yoshiki NAKAMURA and Michico IWATA in Journal of Japanese Environmental Engineering No. 743, p. 21-28, January 2018", and "Visibility evaluation method considering disability glare using luminance contrast evaluation graph" by Yoko KATO, and Yoshiki NAKAMURA in Illuminating Engineering Institute of Japan, Volume 103, No. 2, pp. 67-74, February 2019". In Fig. 4, a region below the visibility threshold curve L is a luminance contrast region where the visual target is visible the test subject, and a region above the visibility threshold curve L is a luminance contrast region where the visual target is not visible to the test subject.

The visibility threshold curve L illustrated in Fig. 4 is expressed by function $LC_{th}$.

$$LC_{th} = LC_{th0} + \Delta C_Y + \Delta C_X + \Delta C_Z \qquad (2)$$

In above Equation (2), $LC_{th0}$ is a term for the luminance contrast visibility threshold for cases in which a body equivalent to a background luminance of a visual target has been placed at a periphery of the visual target having a spectral distribution of tristimulus values imparted to the test subject equivalent to the spectral distribution of standard illuminant D65 of the International Commission on Illumination (CIE) (hereafter referred to as D65). $\Delta C_Y$ is a term to reflect an incremental threshold from superposition of the tristimulus values of D65 for cases in which a body having a different luminance to the background luminance of the visual target is placed at the periphery of the visual target. $\Delta C_X$ and $\Delta C_Z$ are visibility functions to approximate to points (visibility threshold data) that take into account a change in visual acuity due to color superposition, and are functions that express that visual acuity is different depending on the spectral distribution a visual target. More specifically, they are terms to reflect incremental thresholds from superposition in cases in which a body of spectral distribution that imparts tristimulus values different to the spectral distribution of D65 is placed at the periphery of the visual target.

The calculation unit 101 calculates the tristimulus values X, Y, Z for each pixel from the two-dimensional spectral radiance image data of the visual object. The spectral distribution is taken into account when calculating the evaluation function $F_{VIS}$ via the tristimulus values. The term $LC_{th0}$ on the right hand side of Equation (2) is a function of variable Y and parameter $\alpha$. The term $\Delta C_Y$ is a function of variable Y and parameter $a_Y$. The term $\Delta C_X + \Delta C_Z$ are functions of variables X and Z, and parameters bx and bz. Note that parameters $\alpha$, $a_Y$, bx, bz are parameters expressing the visual acuity of the test subject, and are found by fitting each function term to the luminance contrast visibility threshold obtained under the respective examination conditions described above. Namely, the parameters $\alpha$, $a_Y$, bx, bz have unique values peculiar to each test subject.

The evaluation value may be calculated for a specified part region of a visual object. When doing so, the region for calculation may be a part region of the visual object specified using the results of edge detection on the visual object, and may be specified by an examiner or the test subject.

When calculating the evaluation value, the calculation unit 101 uses the spectral distribution data resulting from application (multiplication) of a filter having a given spectral transmittance to the two-dimensional spectral radiance image data of the visual object, and calculates a Y tristimulus value for each pixel and calculates a luminance contrast $LC_{obj}$ of the visual object. Employing the spectral distribution data applied with the filter enables design of an optical member optimized for each test subject, namely, design of a filter having a spectral transmittance optimized for each test subject.

When calculating the evaluation value, the calculation unit 101 calculates the evaluation value for a given region within the two-dimensional spectral radiance image. For example, a region may be set for the body within the two-dimensional spectral radiance image, and a region may be set so as to include a boundary between bodies.

The calculation unit 101 first calculates the evaluation value for the spectral distribution of the visual object in a state in which a filter having given spectral transmittance is not applied, and extracts a point from the visual object where the evaluation value is maximized (first point). Reference here to a point means a position on the two-dimensional spectral radiance image. The calculation unit 101 then calculates the evaluation value for the spectral distribution of the visual object in a state in which the filter having the given spectral transmittance has been applied, and extracts a point from the visual object where the evaluation value is maximized (second point). In cases in which the first point and the second point are different, the calculation unit 101 calculates the evaluation value for the first point.

The spectral transmittance optimization unit 102 uses the evaluation value calculated by the calculation unit 101 to optimize the spectral transmittance of the optical member for applicability to the test subject having the luminance contrast visibility threshold $LC_{th}$ as expressed by the denominator of the evaluation function $F_{VIS}$. For example, the spectral transmittance optimization unit 102 decides a filter having a spectral transmittance so as to maximize the evaluation value. For example, the spectral transmittance optimization unit 102 may decide the spectral transmittance for which the evaluation value is the maximum by employing a specific numerical optimization algorithm. For example, a solver function of Excel (registered trademark) software from Microsoft Corporation may be employed as the specific numerical optimization algorithm. Moreover, for example, the spectral transmittance optimization unit 102 may decide the spectral transmittance so as to maximize the evaluation value based on results of machine learning.

Note that even when the spectral transmittance can be decided so as to maximize the evaluation value, this does not necessarily mean that this spectral transmittance can be realized as this because of the combination of dyes actually available. The spectral transmittance optimization unit 102 may accordingly decide the spectral transmittance so as to maximize the evaluation value from out of spectral transmittances generated by finite combinations of pre-prepared dyes.

Following description is regarding a specific example of processing by the calculation unit 101 and the spectral transmittance optimization unit 102. For example, in cases in which 10 types of filter have been prepared that each have different respective spectral transmittances, the calculation unit 101 may apply the respective spectral transmittances to the two-dimensional spectral radiance image data of the visual object to obtain corresponding pairs of the 10 individual $LC_{obj}$ and $LC_{th}$. The spectral transmittance optimization unit 102 employs the respective pairs of the 10 individual $LC_{obj}$ and $LC_{th}$ calculated by the calculation unit 101 to calculate the evaluation value, and selects the pair of $LC_{obj}$ and $LC_{th}$ so as to maximize the evaluation value. By deciding the filter having the spectral transmittance corresponding to the pair of $LC_{obj}$ and $LC_{th}$ that maximizes the evaluation value, the spectral transmittance optimization unit 102 optimizes the spectral transmittance of the filter for applicability to the test subject.

The information processing device 10 according to the present exemplary embodiment has such a configuration, and so is able to design an optimal optical member for each test subject using an evaluation function derivable from the luminance contrast visibility threshold of the test subject and luminance contrast of the visual object.

Next, following description is regarding operation of the information processing device 10.

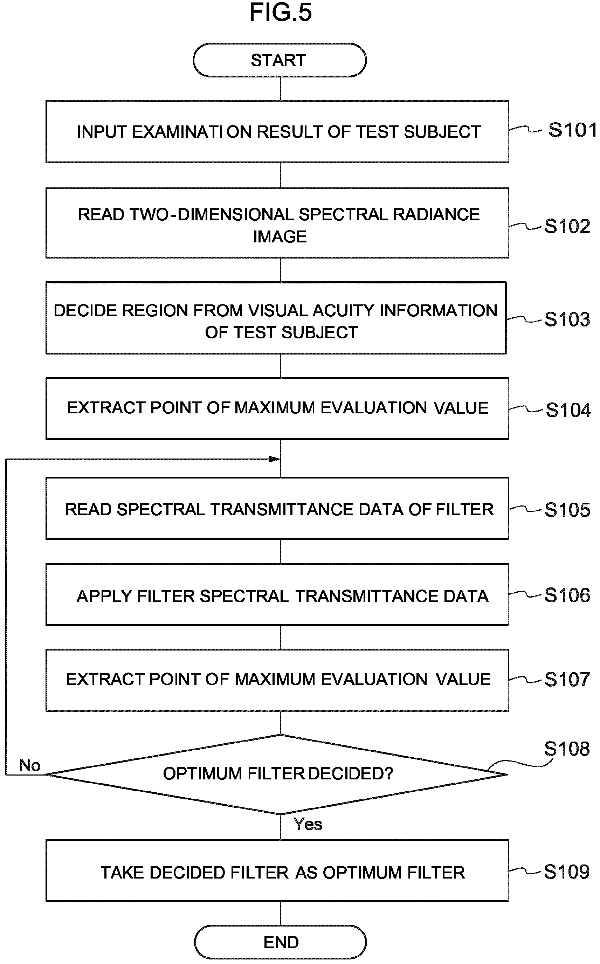

Fig. 5 is a flowchart illustrating a flow of optical member design processing by the information processing device 10. The optical member design processing is performed by the CPU 11 reading the optical member design program from the ROM 12 or the storage 14, and expanding and executing the program in the RAM 13.

The CPU 11 first inputs examination results for the test subject, namely visibility threshold data for the test subject, calculates the luminance contrast visibility threshold from this data, and calculates parameters $\alpha$, $a_Y$, bx, bz to express the visual acuity of the test subject by fitting the function $LC_{th}$ to the luminance contrast visibility thresholds (step S101). In addition to input of the visibility threshold data, the CPU 11 acquires the two-dimensional spectral radiance image of spectral radiance data measured for each pixel (step S102). Acquiring the two-dimensional spectral radiance image enables output of a RGB image, a log-average luminance image, and a luminance contrast image.

Continuing from step S102, the CPU 11 decides a region to calculate the evaluation value in the two-dimensional spectral radiance image data based on the examination result of the test subject (step S103). The CPU 11 may decide the region based on instruction from the examiner, and may decide the region based on image processing performed on the two-dimensional spectral radiance image.

Continuing from step S103, the CPU 11 extracts the point with the maximum evaluation value in the region decided at step S103 (step S104). Namely, the CPU 11 first performs calculation of the evaluation value for a case in which no filter is applied, and extracts the point with the maximum evaluation value therein. The CPU 11 calculates the evaluation value based on the above Equation (1) and Equation (2).

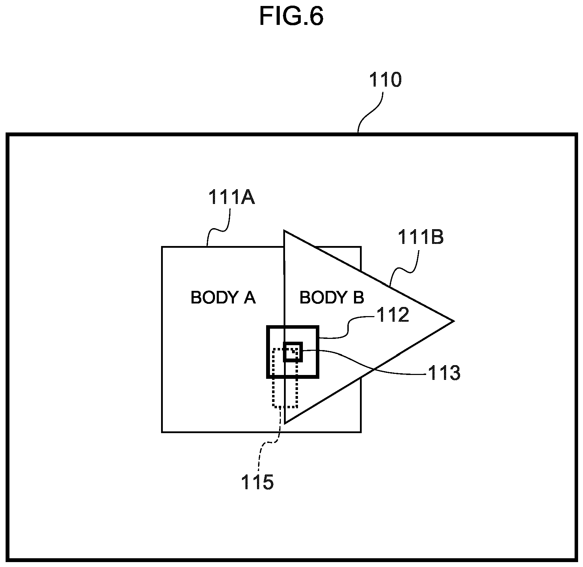

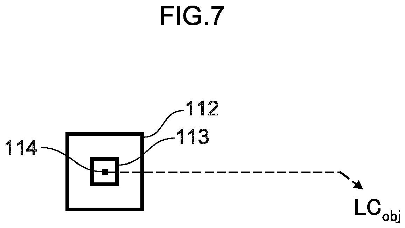

Fig. 6 and Fig. 7 are diagrams to explain the computational processing of the evaluation function $F_{VIS}$ by the CPU 11. A two-dimensional spectral radiance image 110 is illustrated in Fig. 6. A rectangular body A111A and a triangular body B 111B are imaged in the two-dimensional spectral radiance image 110. The method of calculating the luminance contrast $LC_{obj}$ of the visual object is as disclosed in "Study on visibility evaluation of circular visual targets for sighted subjects - research on visibility evaluation method using a luminance contrast evaluation graph assuming subjects with poor vision, Part 1" by Yoko KATO, Yoshiki NAKAMURA and Michico IWATA in Journal of Japanese Environmental Engineering No. 743, p. 21-28, January 2018", and employs an N filter represented by a 9 × 9 grid. The reference numeral 112 indicates a 9 pixel × 9 pixel region where the N filter is applied, and reference numeral 113 indicates 3 pixel × 3 pixel of the N filter corresponding to a region described as a visual object region. The regions of the N filter other than at reference numeral 113 are a region described as the background. The CPU 11 performs an N filter convolutional operation on the log of the pixel luminance values for the background region 112 and the visual object region 113, and calculates the luminance contrast $LC_{obj}$ of a center point (position) 114 of the N filter. Thus the evaluation value is calculated as the value for the center point (position) of the N filter.

Although the evaluation value may be calculated for all of the points of the two-dimensional spectral radiance image 110, the efficiency of processing can be raised by limiting the calculation target. Reference numeral 115 indicates an example of a region where the evaluation value is calculated. The region 115 may be decided by being specified by the examiner or the test subject, and may be decided by performing edge detection in the two-dimensional spectral radiance image 110. For example, in cases in which the body A111A is a red body and the body B111B is a yellow body, the test subject may set the boundary between the body A111A and the body B111B when the test subject thinks they would like to see the boundary between the body A111A and the body B111B clearly. The evaluation value may then be calculated with the boundary portion set by the test subject as the calculation target. In the example of the two-dimensional spectral radiance image 110 illustrated in Fig. 6, the region 115 is decided by performing edge detection of the boundary between the body A111A and the body B111B, or by being specified by the test subject or examiner.

Note that the evaluation value region 115 is not limited to the example illustrated in Fig. 6. For example, in cases in which there is a desire to show the body A111A vividly to the test subject, then the region 115 may be decided so as to only contain the body A111A.

Moreover similarly, although a luminance contrast $LC_{obj}$ may be calculated for all regions of the two-dimensional spectral radiance image 110, the region of the luminance contrast $LC_{obj}$ may also be restricted. Restricting the region of the luminance contrast $LC_{obj}$ enables more efficient processing.

Continuing from step S104, the CPU 11 reads spectral transmittance data of a filter for application to the two-dimensional spectral radiance image 110 (step S105). In cases in which spectral transmittance data are prepared for plural filters, the CPU 11 may read in any of the spectral transmittance data.

Continuing from step S105, the CPU 11 applies the spectral transmittance data read at step S105 to the two-dimensional spectral radiance image 110 (step S106). Applying the spectral transmittance data to the two-dimensional spectral radiance image 110 enables a filter-applied state to be output for a RGB image, a log-average luminance image, and a luminance contrast image.

Continuing from step S106, the CPU 11 extracts a point with the maximum evaluation value in the region decided at step S103 from the two-dimensional spectral radiance image 110 applied with the spectral transmittance data (step S107). The CPU 11 calculates the evaluation value based on the above Equation (1) and Equation (2).

Continuing from step S107, the CPU 11 compares the point with the maximum evaluation value extracted at step S107 against the evaluation value for different spectral transmittance data, and determines whether or not the optimum filter can be decided (step S108). The CPU 11 determines whether or not the optimum filter can be decided by determining whether or not the evaluation value is the maximum. For example, the CPU 11 sequentially reads in the spectral transmittance data for all filters, and takes the filter with the spectral transmittance resulting in the maximum evaluation value as the optimum filter. Alternatively, for example, the CPU 11 takes the filter with the spectral transmittance resulting in the maximum evaluation value from out of spectral transmittances generated by combining pre-prepared dyes as the optimum filter.

Note that in cases in which the point extracted at step S104 is different from the point extracted at step S107, the evaluation value at the same point as the point extracted at step S104 is employed for comparison.

The CPU 11 repeatedly performs the processing from step S105 to step S108 until the optimum filter can be decided. Then when determined that the optimum filter has been decided (step S108: Yes), the CPU 11 takes the decided filter as the optimum filter for the test subject (step S109).

By executing this cycle of processing, the information processing device 10 according to the present exemplary embodiment is able to use an evaluation function derivable from the luminance contrast visibility threshold of the test subject and from the luminance contrast of the visual object to design the optimal optical member function for each of the test subjects.

Although in the exemplary embodiment described above the information processing device 10 employs the luminance contrast visibility threshold as the visibility threshold for the test subject, and employs the luminance contrast of the visual object calculated from the spectral radiance of the visual object as a parameter related to the visual object, the present disclosure is not limited so such an example. For example, the information processing device 10 may employ a visibility threshold in color space as the visibility threshold of the test subject, and may employ color information of the visual object as the parameter related to the visual object. The color information of the visual object may, for example, be derived from the spectral radiance of the visual object.

Note that the optical member design processing executed in the exemplary embodiment described above by the CPU reading in software (a program) may be executed by various processors other than the CPU. Examples of such processors include programmable logic devices (PLD) that allow circuit configuration to be modified post-manufacture, such as a field-programmable gate array (FPGA), and dedicated electric circuits, these being processors including a circuit configuration custom-designed to execute specific processing, such as an application specific integrated circuit (ASIC). The optical member design processing may be executed by any one of these various types of processors, or may be executed by a combination of two or more of the same type or different types of processors (such as plural FPGAs, or a combination of a CPU and an FPGA). The hardware structure of these various types of processors is more specifically an electric circuit combining circuit elements such as semiconductor elements.

Moreover, although in the above exemplary embodiment an embodiment was described in which a program for the optical member design processing was pre-stored (installed) on in storage or ROM, there is no limitation thereto. The program may be provided in a format stored on a non-transitory recording medium such as a compact disk read only memory (CD-ROM), digital versatile disk read only memory (DVD-ROM), universal serial bus (USB) memory, or the like. Moreover, the program may be provided in a format downloadable from an external device over a network.

Explanation of the Reference Numerals

1 optical member design system
10 information processing device
101 calculation unit
102 spectral transmittance optimization unit

**Claims**

1. An information processing device comprising:

   a calculation unit that calculates an evaluation function using a visibility threshold of a test subject, obtained by performing a visual acuity examination on the test subject, and using a parameter related to a visual object; and
   a spectral transmittance optimization unit that optimizes a spectral transmittance of a filter having a specific spectral transmittance to light from the visual object using the evaluation function when passed through the filter.

2. The information processing device of claim 1, wherein the spectral transmittance optimization unit determines the spectral transmittance of the filter such that a value of the evaluation function is maximized.

3. The information processing device of claim 2, wherein the spectral transmittance optimization unit determines the spectral transmittance such that the value of the evaluation function is maximized from among spectral transmittances generated by combining pre-prepared dyes.

4. The information processing device of claim 2, wherein the spectral transmittance optimization unit determines a spectral transmittance such that the value of the evaluation function is maximized using a specific numerical optimization algorithm.

5. The information processing device of claim 1, wherein the calculation unit calculates the evaluation function for a specified part region of the visual object.

6. The information processing device of claim 5, wherein the calculation unit calculates the evaluation function for a part region of the visual object as specified by a result of performing edge detection on the visual object.

7. The information processing device of claim 5, wherein the spectral transmittance optimization unit optimizes the spectral transmittance of the filter using the evaluation function when passed through the filter at a point in the region where a value of the evaluation function when not passed through the filter is maximized.

8. The information processing device of claim 7, wherein, in a case in which a first point that is a point in the region where the value of the evaluation function when not passed through the filter is maximized differs from a second point that is a point in the region where a value of the evaluation function when passed through the filter is maximized, the spectral transmittance optimization unit optimizes the spectral transmittance of the filter using the evaluation function at the first point.

9. The information processing device of any one of claim 1 to claim 8, wherein the visibility threshold of the test subject is a luminance contrast visibility threshold.

10. The information processing device of claim 9, wherein the luminance contrast visibility threshold is generated by finding a boundary between a luminance contrast region where a visual target is visible to the test subject and a luminance contrast region where the visual target is not visible to the test subject in a coordinate system indicating a correlation between a luminance contrast value between a luminance of an attention portion and a luminance of a background portion, and a luminance average value of the visual target including the attention portion and the background portion.

11. The information processing device of claim 10, wherein the boundary is generated by finding a visibility function that approximates to one which takes visibility changes into account due to superposition of color of visual targets in the coordinate system.

12. The information processing device of any one of claim 1 to claim 11, wherein the parameter related to the visual object is a luminance contrast of the visual object calculated from spectral radiance of the visual object.

13. An information processing method of processing executed by a computer, the processing comprising:

    calculating an evaluation function using a visibility threshold of a test subject, obtained by performing a visual acuity examination on the test subject, and using a parameter related to a visual object; and
    optimizing a spectral transmittance of a filter having a spectral transmittance to light from the visual object using the evaluation function when passed through the filter.

14. A computer program that causes a computer to execute processing, the processing comprising:

    calculating an evaluation function using a visibility threshold of a test subject, obtained by performing a visual acuity examination on the test subject, and using a parameter related to a visual object; and
    optimizing a spectral transmittance of a filter having a spectral transmittance to light from the visual object using the evaluation function when passed through the filter.

# FIG.1

<u>1</u>

```
┌─────────────────────────────────┐
│          INPUT DEVICE           │ ～20
└─────────────────────────────────┘
                 │
┌─────────────────────────────────┐
│   INFORMATION PROCESSING        │ ～10
│           DEVICE                │
└─────────────────────────────────┘
                 │
┌─────────────────────────────────┐
│         DISPLAY DEVICE          │ ～30
└─────────────────────────────────┘
```

# FIG.2

# FIG.3

10

101 — CALCULATION UNIT

102 — SPECTRAL TRANSMITTANCE OPTIMIZATION UNIT

# FIG.4

# FIG.5

```
              ( START )
                  │
                  ▼
┌─────────────────────────────────────────┐
│ INPUT EXAMINATION RESULT OF TEST SUBJECT │ ～ S101
└─────────────────────────────────────────┘
                  │
                  ▼
┌─────────────────────────────────────────┐
│  READ TWO-DIMENSIONAL SPECTRAL RADIANCE  │ ～ S102
│                 IMAGE                    │
└─────────────────────────────────────────┘
                  │
                  ▼
┌─────────────────────────────────────────┐
│ DECIDE REGION FROM VISUAL ACUITY INFORMATION │ ～ S103
│             OF TEST SUBJECT              │
└─────────────────────────────────────────┘
                  │
                  ▼
┌─────────────────────────────────────────┐
│  EXTRACT POINT OF MAXIMUM EVALUATION VALUE │ ～ S104
└─────────────────────────────────────────┘
                  │
                  ▼
┌─────────────────────────────────────────┐
│  READ SPECTRAL TRANSMITTANCE DATA OF FILTER │ ～ S105
└─────────────────────────────────────────┘
                  │
                  ▼
┌─────────────────────────────────────────┐
│   APPLY FILTER SPECTRAL TRANSMITTANCE DATA │ ～ S106
└─────────────────────────────────────────┘
                  │
                  ▼
┌─────────────────────────────────────────┐
│  EXTRACT POINT OF MAXIMUM EVALUATION VALUE │ ～ S107
└─────────────────────────────────────────┘
                  │
                  ▼
     No        ◇ OPTIMUM FILTER DECIDED? ◇   S108
                  │ Yes
                  ▼
┌─────────────────────────────────────────┐
│   TAKE DECIDED FILTER AS OPTIMUM FILTER  │ ～ S109
└─────────────────────────────────────────┘
                  │
                  ▼
               ( END )
```

# FIG.6

110

111A

111B

BODY A    BODY B

112

113

115

# FIG.7

## INTERNATIONAL SEARCH REPORT

International application No.

**PCT/JP2021/038456**

### A. CLASSIFICATION OF SUBJECT MATTER

*A61B 3/028*(2006.01)i
FI: A61B3/028

According to International Patent Classification (IPC) or to both national classification and IPC

### B. FIELDS SEARCHED

Minimum documentation searched (classification system followed by classification symbols)

A61B3/028

Documentation searched other than minimum documentation to the extent that such documents are included in the fields searched

Published examined utility model applications of Japan 1922-1996
Published unexamined utility model applications of Japan 1971-2021
Registered utility model specifications of Japan 1996-2021
Published registered utility model applications of Japan 1994-2021

Electronic data base consulted during the international search (name of data base and, where practicable, search terms used)

### C. DOCUMENTS CONSIDERED TO BE RELEVANT

| Category* | Citation of document, with indication, where appropriate, of the relevant passages | Relevant to claim No. |
|---|---|---|
| X | JP 2019-517850 A (ESSILOR INT.) 27 June 2019 (2019-06-27) paragraphs [0063]-[0422] | 1-14 |
| A | WO 2010/147089 A1 (NTT DOCOMO, INC.) 23 December 2010 (2010-12-23) entire text, all drawings | 1-14 |
| A | JP 2005-207940 A (ASAHI GLASS CO., LTD.) 04 August 2005 (2005-08-04) entire text, all drawings | 1-14 |
| A | WO 2018/012334 A1 (VISUAL TECHNOLOGY LABORATORY INC.) 18 January 2018 (2018-01-18) entire text, all drawings | 1-14 |

☐ Further documents are listed in the continuation of Box C.  ☑ See patent family annex.

| | |
|---|---|
| * Special categories of cited documents:<br>"A" document defining the general state of the art which is not considered to be of particular relevance<br>"E" earlier application or patent but published on or after the international filing date<br>"L" document which may throw doubts on priority claim(s) or which is cited to establish the publication date of another citation or other special reason (as specified)<br>"O" document referring to an oral disclosure, use, exhibition or other means<br>"P" document published prior to the international filing date but later than the priority date claimed | "T" later document published after the international filing date or priority date and not in conflict with the application but cited to understand the principle or theory underlying the invention<br>"X" document of particular relevance; the claimed invention cannot be considered novel or cannot be considered to involve an inventive step when the document is taken alone<br>"Y" document of particular relevance; the claimed invention cannot be considered to involve an inventive step when the document is combined with one or more other such documents, such combination being obvious to a person skilled in the art<br>"&" document member of the same patent family |

| Date of the actual completion of the international search | Date of mailing of the international search report |
|---|---|
| **20 December 2021** | **28 December 2021** |

| Name and mailing address of the ISA/JP | Authorized officer |
|---|---|
| **Japan Patent Office (ISA/JP)**<br>**3-4-3 Kasumigaseki, Chiyoda-ku, Tokyo 100-8915**<br>**Japan** | |
| | Telephone No. |

Form PCT/ISA/210 (second sheet) (January 2015)

**INTERNATIONAL SEARCH REPORT**
Information on patent family members

International application No.

**PCT/JP2021/038456**

| Patent document cited in search report | | | Publication date (day/month/year) | Patent family member(s) | | | Publication date (day/month/year) |
|---|---|---|---|---|---|---|---|
| JP | 2019-517850 | A | 27 June 2019 | US paragraphs [0111]-[0550]<br>WO<br>CN<br>KR | 2019/0212581<br>2017/194898<br>109073918<br>10-2019-0005847 | A1<br>A1<br>A<br>A | |
| WO | 2010/147089 | A1 | 23 December 2010 | US entire text, all drawings<br>CN<br>KR | 2012/0154394<br>102461192<br>10-2012-0023790 | A1<br>A<br>A | |
| JP | 2005-207940 | A | 04 August 2005 | (Family: none) | | | |
| WO | 2018/012334 | A1 | 18 January 2018 | US entire text, all drawings<br>KR<br>CN | 2019/0239741<br>10-2019-0008919<br>109640786 | A1<br>A<br>A | |

Form PCT/ISA/210 (patent family annex) (January 2015)

## REFERENCES CITED IN THE DESCRIPTION

### Patent documents cited in the description

- WO 2018012334 A

### Non-patent literature cited in the description

- **YOKO KATO ; YOSHIKI NAKAMURA ; MICHICO IWATA.** Study on visibility evaluation of circular visual targets for sighted subjects - research on visibility evaluation method using a luminance contrast evaluation graph assuming subjects with poor vision. *Journal of Japanese Environmental Engineering,* July 2018, vol. 83 (749), 565-572
- **YOKO KATO ; YOSHIKI NAKAMURA ; MICHICO IWATA.** Study on visibility evaluation of circular visual targets for sighted subjects - research on visibility evaluation method using a luminance contrast evaluation graph assuming subjects with poor vision, Part 1. *Journal of Japanese Environmental Engineering,* January 2018, (743), 21-28
- **YOKO KATO ; YOSHIKI NAKAMURA.** Visibility evaluation method considering disability glare using luminance contrast evaluation graph. *Illuminating Engineering Institute of Japan,* February 2019, vol. 103 (2), 67-74